# EUROPEAN PATENT SPECIFICATION

(11) **EP 4 182 318 B1**
(45) Date of publication and mention of the grant of the patent: **27.08.2025**
(21) Application number: 21749456.6
(22) Date of filing: 08.07.2021
(51) Int. Cl.: C07D 487/06, A61P 35/00, A61K 31/55

(54) **SOLID STATE FORMS OF RUCAPARIB SALTS**
FESTE FORMEN VON RUCAPARIBSALZEN
FORMES À L'ÉTAT SOLIDE DE SELS DE RUCAPARIB

(30) Priority: 14.07.2020 US 202063051454 P
(43) Date of publication of application: 24.05.2023
(73) Proprietor: Assia Chemical Industries Ltd., Tel Aviv 6944020 (IL)
(72) Inventor: SAHNIC , Damir, 10104 Zagreb (HR); TRAVANCIC , Valentina, 10 000 Zagreb (HR); SAMEC, Dijana Skalec, 10450 Jastrebarsko (HR)
(74) Representative: D Young & Co LLP
(86) International application number: PCT/US2021/040785
(87) International publication number: WO 2022/015557

(56) References cited:
- WO-A1-2018/140377

## Description

### FIELD OF THE DISCLOSURE

The present disclosure encompasses solid state forms of Rucaparib and of Rucaparib salts, and pharmaceutical compositions thereof.

### BACKGROUND OF THE DISCLOSURE

Rucaparib chemical name is 8-fluoro-2-{4-[(methylamino)methyl]phenyl}-1,3,4,5-tetrahydro-6*H*-azepino[5,4,3-*cd*]indol-6-one, having the following chemical structure:

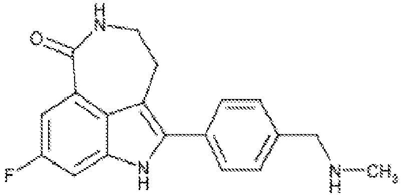

Rucaparib S-Camsylate chemical name is 8-fluoro-2-{4-[(methylamino)methyl]phenyl }-1,3,4,5-tetrahydro-6*H*-azepino[5,4,3-cd]indol-6-one ((1S,4R)-7,7 dimethyl-2-oxobicyclo[2.2.1]hept-1-yl) methane-sulfonic acid salt, having the following chemical structure:

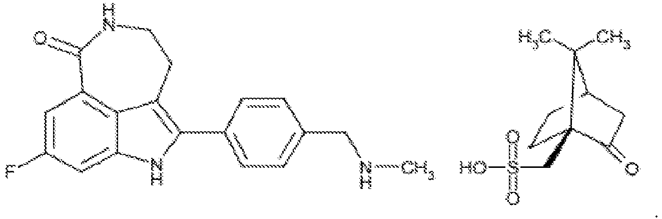

Rucaparib Tosylate chemical name is 8-fluoro-2-{4-[(methylamino)methyl]phenyl}-1,3,4,5-tetrahydro-6*H*-azepino[5,4,3-*cd*]indol-6-oneTosylate, having the following chemical structure:

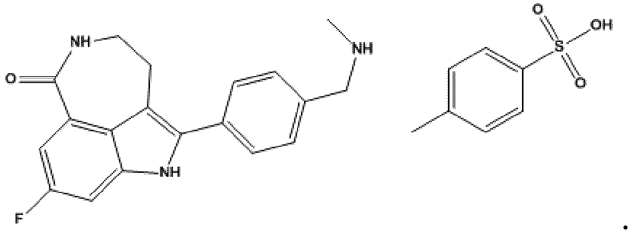

Rucaparib is an inhibitor of the mammalian poly-adenosine 5'-diphospho-ribose polymerase (PARP) enzyme, indicated as monotherapy for the treatment of patients with deleterious BRCA mutation (germline and/or somatic) associated advanced ovarian cancer who have been treated with two or more chemotherapies. It was approved by the FDA as RUBRACA (Rucaparib Camsylate).

The compound is described in U.S. Patent No. 6,495,541. A process for its preparation is described in U.S. Patent No. 7,323,562, as well as in Org. Process Res. Dev., 2012, 16 (12), pp 1897-1904. U.S. Patent Publication No. 2004/0248879 also describes the following salts of Rucaparib: HCl, Mesylate, Phosphate, Glucuronate, Tartrate, Gluconate and Acetate. U.S. Patent No. 7,268,126 describes crystalline forms and an amorphous form of Rucaparib Phosphate. U.S. Patent No. 8,754,072 describes crystalline Camsylate and Maleate salts of Rucaparib. U.S. Patent No. 9,045,487 describes Camsylate and Maleate salts of Rucaparib.

U.S. Patent Publication No. 2019/0389871 describes solid state forms of Rucaparib and Rucaparib salts.

International Publication No. WO 2018/140377 describes several solid state forms of Rucaparib and Rucaparib salts, including Rucaparib tosylate Forms I-V and a mixture of Forms V and VI.

Rucaparib is administrated in a high-load dose, of 600 mg twice daily; available tablets strength are 200 mg, 250 mg, and 300 mg.

International Publication No. WO 2016/028689 describes a tablet of Rucaparib Camsylate in certain API amount. According to this application, at high drug loading, the contribution of the physical properties of the active pharmaceutical ingredient ("API") to the manufacturability of a formulation is predominant. According to the applicant, high drug loading requires compressibility properties which are contributed due to the API. In that application, it is stated that Rucaparib Camsylate has advantageous properties with respect to compressibility and that it is possible to manufacture tablets thereof with a load of 45% w/w or more.

Polymorphism, the occurrence of different crystalline forms, is a property of some molecules and molecular complexes. A single molecule may give rise to a variety of polymorphs having distinct crystal structures and physical properties like melting point, thermal behaviors (e.g., measured by thermogravimetric analysis - "TGA", or differential scanning calorimetry - "DSC"), X-ray diffraction (XRD) pattern, infrared absorption fingerprint, and solid state (¹³C) NMR spectrum. One or more of these techniques may be used to distinguish different polymorphic forms of a compound.

Different salts and solid state forms (including solvated forms) of an active pharmaceutical ingredient may possess different properties. Such variations in the properties of different salts and solid state forms and solvates may provide a basis for improving formulation, for example, by facilitating better processing or handling characteristics, changing the dissolution profile in a favorable direction, or improving stability (polymorph as well as chemical stability) and shelf-life. These variations in the properties of different salts and solid state forms may also offer improvements to the final dosage form, for instance, if they serve to improve bioavailability. Different salts and solid state forms and solvates of an active pharmaceutical ingredient may also give rise to a variety of polymorphs or crystalline forms, which may in turn provide additional opportunities to assess variations in the properties and characteristics of a solid active pharmaceutical ingredient.

Discovering new solid state forms and solvates of a pharmaceutical product may yield materials having desirable processing properties, such as ease of handling, ease of processing, storage stability, and ease of purification or as desirable intermediate crystal forms that facilitate conversion to other polymorphic forms. New solid state forms of a pharmaceutically useful compound can also provide an opportunity to improve the performance characteristics of a pharmaceutical product. It enlarges the repertoire of materials that a formulation scientist has available for formulation optimization, for example by providing a product with different properties, e.g., a different crystal habit, higher crystallinity, or polymorphic stability, which may offer better processing or handling characteristics, improved dissolution profile, or improved shelf-life (chemical/physical stability). For at least these reasons, there is a need for additional solid state forms (including solvated forms) of Rucaparib.

### SUMMARY OF THE DISCLOSURE

The present disclosure provides solid state forms of Rucaparib and of Rucaparib salts, processes for preparation thereof, and pharmaceutical compositions thereof. These solid state forms can be used to prepare other forms of Rucaparib, Rucaparib salts and solid state forms thereof. The solid state forms of Rucaparib and of Rucaparib salts can be used to prepare other solid state forms of Rucaparib or other solid state forms of salts of Rucaparib.

The present disclosure provides solid state forms of Rucaparib and of Rucaparib salts for use in the preparation of pharmaceutical compositions and/or formulations for use in medicine, preferably for the treatment of cancer.

The present disclosure also encompasses the use of solid state forms of Rucaparib and of Rucaparib salts of the present disclosure for the preparation of pharmaceutical compositions and/or formulations.

In another aspect, the present disclosure provides pharmaceutical compositions and/or pharmaceutical formulations comprising any one or a combination of the solid state forms of Rucaparib and/or of Rucaparib salts according to the present disclosure.

In yet another embodiment, the present disclosure encompasses pharmaceutical formulations comprising any one or a combination of the described solid state forms of Rucaparib and/or of Rucaparib salts, or pharmaceutical compositions comprising them and at least one pharmaceutically acceptable excipient.

The present disclosure comprises processes for preparing the above mentioned pharmaceutical formulations. The processes comprise combining any one of or a combination of the described solid state forms of Rucaparib and/or of Rucaparib salts; or a pharmaceutical composition comprising them with at least one pharmaceutically acceptable excipient.

The solid state forms as defined herein and the pharmaceutical compositions or formulations of solid state forms of Rucaparib and/or Rucaparib salts may be used as medicaments, particularly for the treatment of cancer.

The present disclosure also provides methods of treating cancer comprising administering a therapeutically effective amount of any one or a combination of the solid state forms of Rucaparib and/or of Rucaparib salts of the present disclosure, or at least one of the above pharmaceutical compositions or formulations, to a subject suffering from cancer, or otherwise in need of the treatment.

The present disclosure also provides the uses of solid state forms of Rucaparib and of Rucaparib salts of the present disclosure, or at least one of the above pharmaceutical compositions or formulations, for the manufacture of medicaments for treating cancer.

The present disclosure also provides methods of treating cancer comprising administering a therapeutically effective amount of any one or a combination of the solid state forms of Rucaparib and/or solid state forms of salts of Rucaparib of the present disclosure, or at least one of the above pharmaceutical compositions and/or formulations, to a subject in need of the treatment.

### BRIEF DESCRIPTION OF THE DRAWINGS

Figure 1 shows a characteristic X-ray powder diffraction pattern (XRPD) of Rucaparib Tosylate Form VII.
Figure 2 shows a characteristic X-ray powder diffraction pattern of Rucaparib Tosylate Form VIII.
Figure 3 shows a characteristic X-ray powder diffraction pattern of crystalline Form C of Rucaparib base.

### DETAILED DESCRIPTION OF THE DISCLOSURE

The present disclosure encompasses solid state forms of Rucaparib and of Rucaparib salts. Solid state properties of solid state forms of Rucaparib and salts thereof can be influenced by controlling the conditions under which the solid state forms of Rucaparib and of Rucaparib salts are obtained.

A solid state form (or polymorph) may be referred to herein as polymorphically pure or as substantially free of any other solid state (or polymorphic) forms. As used herein in this context, and unless indicated otherwise, the expression "substantially free of any other forms" will be understood to mean that the solid state form contains about 20% (w/w) or less, about 10% (w/w) or less, about 5% (w/w) or less, about 2% (w/w) or less, about 1% (w/w) or less, or about 0% of any other forms of the subject compound as measured, for example, by XRPD. Thus, solid state forms of Rucaparib and salts thereof described herein as substantially free of any other solid state forms would be understood to contain greater than about 80% (w/w), greater than about 90% (w/w), greater than about 95% (w/w), greater than about 98% (w/w), greater than about 99% (w/w), or about 100% of the subject solid state form of Rucaparib or salt thereof. In some embodiments of the disclosure, the described solid state forms of Rucaparib or salts thereof may contain from about 1% to about 20% (w/w), from about 5% to about 20% (w/w), or from about 5% to about 10% (w/w) of one or more other solid state forms of the same Rucaparib or salt thereof.

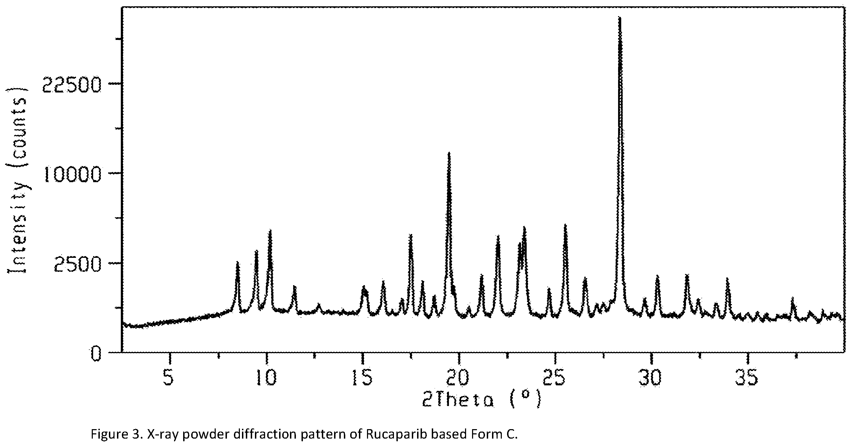

As used herein, crystalline Form C of Rucaparib base refers to Rucaparib base characterized by an X-ray powder diffraction pattern having peaks at 10.5, 16.3, 19.7, 21.4, and 22.2 degrees 2-theta ± 0.2 degrees 2-theta. Alternatively, crystalline Form C of Rucaparib base may refer to Rucaparib base characterized by an X-ray powder diffraction pattern having peaks at 10.5, 16.3, 19.7, 21.4, and 22.2 degrees 2-theta ± 0.2 degrees 2-theta, and also having any one, two, three, four or five additional peaks selected from 8.8, 15.5, 17.7, 18.4, and 26.7 degrees 2-theta ± 0.2 degrees 2-theta. Crystalline Form C or Rucaparib base may be alternatively characterized by an XRPD diffraction pattern substantially as depicted in Figure 3.

Depending on which other solid state forms a comparison is made, the crystalline forms of Rucaparib and salts thereof of the present disclosure have advantageous properties selected from at least one of the following: chemical purity, flowability, solubility, dissolution rate, morphology or crystal habit, stability- such as chemical stability as well as thermal and mechanical stability with respect to polymorphic conversion, stability towards dehydration and/or storage stability, low content of residual solvent, a lower degree of hygroscopicity, flowability, and advantageous processing and handling characteristics such as compressibility, and bulk density. Particularly the crystalline forms of Rucaparib Tosylate according to the present disclosure may have advantageous properties selected from one or more of stability under mechanical stress and solubility or kinetic solubility.

A solid state form, such as a crystal form or an amorphous form, may be referred to herein as being characterized by graphical data "as depicted in" or "as substantially depicted in" a Figure. Such data include, for example, powder X-ray diffractograms and solid state NMR spectra. As is well-known in the art, the graphical data potentially provides additional technical information to further define the respective solid state form (a so-called "fingerprint") which cannot necessarily be described by reference to numerical values or peak positions alone. In any event, the skilled person will understand that such graphical representations of data may be subject to small variations, e.g., in peak relative intensities and peak positions due to certain factors such as, but not limited to, variations in instrument response and variations in sample concentration and purity, which are well known to the skilled person. Nonetheless, the skilled person would readily be capable of comparing the graphical data in the Figures herein with graphical data generated for an unknown crystal form and confirm whether the two sets of graphical data are characterizing the same crystal form or two different crystal forms. A crystal form of Rucaparib or salt thereof referred to herein as being characterized by graphical data "as depicted in" or "as substantially depicted in" a Figure will thus be understood to include any crystal forms of Rucaparib or salt thereof characterized with the graphical data having such small variations, as are well known to the skilled person, in comparison with the Figure.

As used herein, and unless stated otherwise, the term "anhydrous" in relation to crystalline forms of Rucaparib and salts thereof, relates to a crystalline form of Rucaparib or salt thereof which does not include any crystalline water (or other solvents) in a defined, stoichiometric amount within the crystal. Moreover, an "anhydrous" form would typically not contain more than 1% (w/w), of either water or organic solvents as measured for example by TGA.

The term "solvate," as used herein and unless indicated otherwise, refers to a crystal form that incorporates a solvent in the crystal structure. When the solvent is water, the solvate is often referred to as a "hydrate." The solvent in a solvate may be present in either a stoichiometric or in a non-stoichiometric amount.

As used herein, the term "isolated" in reference to solid state forms of Rucaparib and salts thereof of the present disclosure corresponds to a solid state form of Rucaparib or salt thereof that is physically separated from the reaction mixture in which it is formed. The step of isolating solid state forms of Rucaparib or solid state forms of salts of Rucaparib may be performed by crystallization or precipitation.

As used herein, unless stated otherwise, the XRPD measurements are taken using copper Kα radiation wavelength 1.54184 Å. XRPD peaks reported herein are measured using CuK α radiation, λ = 1.5418 Å, at a temperature of 25 ± 3°C.

A thing, e.g., a reaction mixture, may be characterized herein as being at, or allowed to come to "room temperature" or "ambient temperature", often abbreviated as "RT." This means that the temperature of the thing is close to, or the same as, that of the space, e.g., the room or fume hood, in which the thing is located. Typically, room temperature is from about 20°C to about 30°C, or about 22°C to about 27°C, or about 25°C.

The amount of solvent employed in a chemical process, e.g., a reaction or crystallization, may be referred to herein as a number of "volumes" or "vol" or "V." For example, a material may be referred to as being suspended in 10 volumes (or 10 vol or 10V) of a solvent. In this context, this expression would be understood to mean milliliters of the solvent per gram of the material being suspended, such that suspending a 5 grams of a material in 10 volumes of a solvent means that the solvent is used in an amount of 10 milliliters of the solvent per gram of the material that is being suspended or, in this example, 50 mL of the solvent. In another context, the term "v/v" may be used to indicate the number of volumes of a solvent that are added to a liquid mixture based on the volume of that mixture. For example, adding solvent X (1.5 v/v) to a 100 ml reaction mixture would indicate that 150 mL of solvent X was added.

A process or step may be referred to herein as being carried out "overnight." This refers to a time interval, e.g., for the process or step, that spans the time during the night, when that process or step may not be actively observed. This time interval is from about 8 to about 20 hours, or about 10-18 hours, typically about 16 hours.

As used herein, the term "reduced pressure" refers to a pressure that is less than atmospheric pressure. For example, reduced pressure is about 10 mbar to about 50 mbar.

As used herein and unless indicated otherwise, the term "ambient conditions" refer to atmospheric pressure and a temperature of 22-24°C.

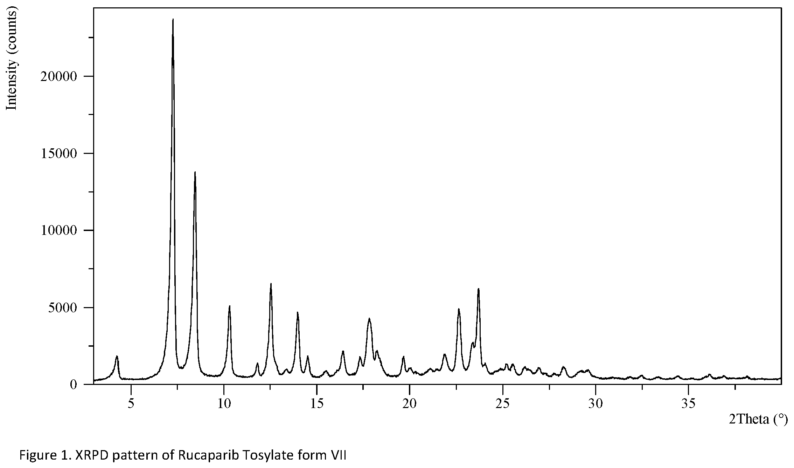

In one embodiment, the present disclosure relates to a crystalline form of Rucaparib Tosylate, designated Form VII. The crystalline Form VII of Rucaparib Tosylate may be characterized by data selected from one or more of the following: an X-ray powder diffraction pattern substantially as depicted in Figure 1; an X-ray powder diffraction pattern having peaks at 4.3, 8.5, 10.3 and 22.7 degrees 2-theta ± 0.2 degrees 2-theta; and combinations of these data.

Crystalline Form VII of Rucaparib Tosylate may be further characterized by an X-ray powder diffraction pattern having peaks at 4.3, 8.5, 10.3 and 22.7 degrees 2-theta ± 0.2 degrees 2-theta and also having any one, two, three or four additional peaks selected from the group consisting of 7.3, 11.8, 14.0 and 17.9 degrees 2-theta ± 0.2 degrees 2-theta.

Crystalline Form VII of Rucaparib Tosylate may be alternatively characterized by an X-ray powder diffraction pattern having peaks at 4.3, 7.3, 8.5, 10.3, 11.8, 14.0, 17.9 and 22.7 degrees 2-theta ± 0.2 degrees 2-theta.

Crystalline Form VII of Rucaparib Tosylate may be characterized by each of the above characteristics alone/or by all possible combinations, e.g., an XRPD pattern having peaks at 4.3, 8.5, 10.3 and 22.7 degrees 2-theta ± 0.2 degrees 2-theta; an XRPD pattern as depicted in Figure 1, and combinations thereof.

Crystalline Form VII of Rucaparib Tosylate may be an anhydrous form.

In one embodiment of the present disclosure, crystalline Form VII of Rucaparib Tosylate is isolated.

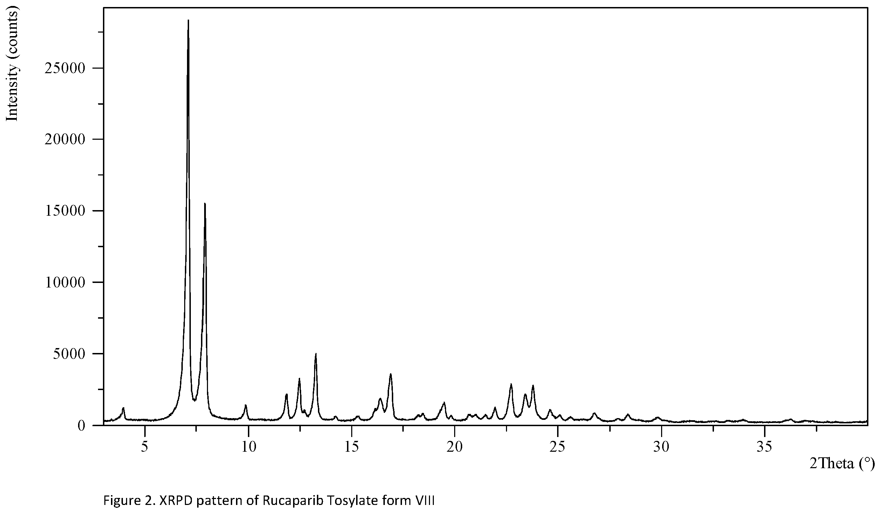

In another embodiment, the present disclosure relates to a crystalline form of Rucaparib Tosylate, designated Form VIII. The crystalline Form VIII of Rucaparib Tosylate may be characterized by data selected from one or more of the following: an X-ray powder diffraction pattern substantially as depicted in Figure 2; an X-ray powder diffraction pattern having peaks at 4.0, 7.9, 13.3, 16.9 and 19.5 degrees 2-theta ± 0.2 degrees 2-theta; and combinations of these data.

Crystalline Form VIII of Rucaparib Tosylate may be characterized by each of the above characteristics alone/or by all possible combinations, e.g., an XRPD pattern having peaks at 4.0, 7.9, 13.3, 16.9 and 19.5 degrees 2-theta ± 0.2 degrees 2-theta; an XRPD pattern as depicted in Figure 2, and combinations thereof.

Crystalline Form VIII of Rucaparib Tosylate may be a methanol solvate.

In one embodiment of the present disclosure, crystalline Form VIII of Rucaparib Tosylate is isolated.

In any aspect or embodiment of the present disclosure, any of the solid state forms of Rucaparib Tosylate described herein may be polymorphically pure or may be substantially free of any other solid state forms of Rucaparib Tosylate. In any aspect or embodiment of the present disclosure, any of the solid state forms of Rucaparib Tosylate may contain: about 20% (w/w) or less, about 10% (w/w) or less, about 5% (w/w) or less, about 2% (w/w) or less, about 1% (w/w) or less, about 0.5% (w/w) or less, about 0.2% (w/w) or less, about 0.1% (w/w) or less, or about 0%, of any other solid state forms of the subject compound, preferably as measured by XRPD. Thus, any of the disclosed crystalline forms of Rucaparib Tosylate described herein may be substantially free of any other solid state forms of Rucaparib Tosylate, and may contain greater than about 80% (w/w), greater than about 90% (w/w), greater than about 95% (w/w), greater than about 98% (w/w), greater than about 99% (w/w), greater than about 99.5% (w/w), greater than about 99.8 wt, or about 100% of the subject crystalline form of Rucaparib Tosylate.

The above solid state forms of Rucaparib and of Rucaparib salts can be used to prepare other solid state forms of Rucaparib or other solid state forms of salts of Rucaparib, such as other solid state forms of Rucaparib Tosylate or Rucaparib camsylate.

The present invention comprises a process for preparing solid state forms of Rucaparib and of Rucaparib salts, comprising preparing any one or a combination of the solid state forms of Rucaparib and of Rucaparib salts of the present invention and converting it to another solid state form of Rucaparib or of Rucaparib salt.

The present disclosure provides solid state forms of Rucaparib and solid state forms of salts of Rucaparib for use in the preparation of pharmaceutical compositions and/or formulations comprising solid state forms of Rucaparib and/or solid state forms of salts of Rucaparib.

The present disclosure also encompasses the use of the solid state forms of Rucaparib and/or solid state forms of salts of Rucaparib of the present disclosure for the preparation of pharmaceutical compositions and/or formulations of solid state forms of Rucaparib and/or solid state forms of salts of Rucaparib.

Pharmaceutical combinations or formulations of the present disclosure contain any one or a combination of the solid state forms of Rucaparib of the present disclosure. In addition to the active ingredient, the pharmaceutical formulations of the present disclosure can contain one or more excipients. Excipients are added to the formulation for a variety of purposes.

In any aspect or embodiment of the disclosure, pharmaceutical compositions or formulations of the present disclosure include solid dosage forms such as tablets, powders, capsules, suppositories, sachets, troches, and lozenges, or liquid dosage forms such as liquid syrups, suspensions, and elixirs. In any aspect or embodiment of the disclosure, the pharmaceutical compositions or formulations of the present disclosure are solid dosage forms such as tablets, powders, capsules, suppositories, sachets, troches, and lozenges. Particularly, the pharmaceutical compositions or formulations of the present disclosure may be for oral administration, more particularly, the pharmaceutical compositions or formulations of the present disclosure may be tablets, powders, capsules, or lozenges, and especially tablets or capsules and more particularly tablets.

Diluents increase the bulk of a solid pharmaceutical composition, and can make a pharmaceutical dosage form containing the composition easier for the patient and caregiver to handle. Diluents for solid compositions include, for example, microcrystalline cellulose (e.g., Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., Eudragit^{®}), potassium chloride, powdered cellulose, sodium chloride, sorbitol, and talc.

Solid pharmaceutical compositions that are compacted into a dosage form, such as a tablet, can include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate, and starch.

The dissolution rate of a compacted solid pharmaceutical composition in the patient's stomach can be increased by the addition of a disintegrant to the composition. Disintegrants include alginic acid, carboxymethylcellulose calcium, carboxymethylcellulose sodium (e.g., Ac-Di-Sol^{®}, Primellose^{®}), colloidal silicon dioxide, croscarmellose sodium, crospovidone (e.g., Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g., Explotab^{®}), and starch.

Glidants can be added to improve the flowability of a non-compacted solid composition and to improve the accuracy of dosing. Excipients that can function as glidants include colloidal silicon dioxide, magnesium trisilicate, powdered cellulose, starch, talc, and tribasic calcium phosphate.

When a dosage form such as a tablet is made by the compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease the release of the product from the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, and zinc stearate.

Flavoring agents and flavor enhancers make the dosage form more palatable to the patient. Common flavoring agents and flavor enhancers for pharmaceutical products that can be included in the composition of the present disclosure include maltol, vanillin, ethyl vanillin, menthol, citric acid, fumaric acid, ethyl maltol, and tartaric acid.

Solid and liquid compositions can also be dyed using any pharmaceutically acceptable colorant to improve their appearance and/or facilitate patient identification of the product and unit dosage level.

In liquid pharmaceutical compositions of the present invention, Rucaparib and any other solid excipients can be dissolved or suspended in a liquid carrier such as water, vegetable oil, alcohol, polyethylene glycol, propylene glycol, or glycerin.

Liquid pharmaceutical compositions can contain emulsifying agents to disperse uniformly throughout the composition an active ingredient or other excipient that is not soluble in the liquid carrier. Emulsifying agents that can be useful in liquid compositions of the present invention include, for example, gelatin, egg yolk, casein, cholesterol, acacia, tragacanth, chondrus, pectin, methyl cellulose, carbomer, cetostearyl alcohol, and cetyl alcohol.

Liquid pharmaceutical compositions of the present invention can also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, xanthan gum and combinations thereof.

Sweetening agents such as sorbitol, saccharin, sodium saccharin, sucrose, aspartame, fructose, mannitol, and invert sugar can be added to improve the taste.

Preservatives and chelating agents such as alcohol, sodium benzoate, butylated hydroxyl toluene, butylated hydroxyanisole, and ethylenediamine tetraacetic acid can be added at levels safe for ingestion to improve storage stability.

According to the present disclosure, a liquid composition can also contain a buffer such as gluconic acid, lactic acid, citric acid, or acetic acid, sodium gluconate, sodium lactate, sodium citrate, or sodium acetate. Selection of excipients and the amounts used can be readily determined by the formulation scientist based upon experience and consideration of standard procedures and reference works in the field.

The solid compositions of the present disclosure include powders, granulates, aggregates, and compacted compositions. The dosages include dosages suitable for oral, buccal, rectal, parenteral (including subcutaneous, intramuscular, and intravenous), inhalant, and ophthalmic administration. Although the most suitable administration in any given case will depend on the nature and severity of the condition being treated, in embodiments the route of administration is oral. The dosages can be conveniently presented in unit dosage form and prepared by any of the methods well-known in the pharmaceutical arts.

Dosage forms include solid dosage forms like tablets, powders, capsules, suppositories, sachets, troches, and lozenges, as well as liquid syrups, suspensions, and elixirs.

The dosage form of the present disclosure can be a capsule containing the composition, such as a powdered or granulated solid composition of the disclosure, within either a hard or soft shell. The shell can be made from gelatin and optionally contain a plasticizer such as glycerin and/or sorbitol, an opacifying agent and/or colorant.

The active ingredient and excipients can be formulated into compositions and dosage forms according to methods known in the art.

A composition for tableting or capsule filling can be prepared by wet granulation. In wet granulation, some or all of the active ingredients and excipients in powder form are blended and then further mixed in the presence of a liquid, typically water, that causes the powders to clump into granules. The granulate is screened and/or milled, dried, and then screened and/or milled to the desired particle size. The granulate can then be tableted, or other excipients can be added prior to tableting, such as a glidant and/or a lubricant.

A tableting composition can be prepared conventionally by dry blending. For example, the blended composition of the actives and excipients can be compacted into a slug or a sheet and then comminuted into compacted granules. The compacted granules can subsequently be compressed into a tablet.

As an alternative to dry granulation, a blended composition can be compressed directly into a compacted dosage form using direct compression techniques. Direct compression produces a more uniform tablet without granules. Excipients that are particularly well suited for direct compression tableting include microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the present disclosure can include any of the aforementioned blends and granulates that were described with reference to tableting, but they are not subjected to a final tableting step.

A pharmaceutical formulation of Rucaparib can be administered. Rucaparib may be formulated for administration to a mammal, in embodiments to a human, by injection. Rucaparib can be formulated, for example, as a viscous liquid solution or suspension, such as a clear solution, for injection. The formulation can contain one or more solvents. A suitable solvent can be selected by considering the solvent's physical and chemical stability at various pH levels, viscosity (which would allow for syringeability), fluidity, boiling point, miscibility, and purity. Suitable solvents include alcohol USP, benzyl alcohol NF, benzyl benzoate USP, and Castor oil USP. Additional substances can be added to the formulation such as buffers, solubilizers, and antioxidants, among others. Ansel et al., Pharmaceutical Dosage Forms and Drug Delivery Systems, 7th ed.

The present disclosure comprises processes for preparing the above mentioned pharmaceutical formulations. The processes comprise combining any one or a combination of the solid state forms of Rucaparib and/or solid state forms of salts of Rucaparib of the present disclosure with at least one pharmaceutically acceptable excipient.

The solid state forms of Rucaparib and/or solid state forms of salts of Rucaparib and the pharmaceutical compositions of solid state forms of Rucaparib and/or solid state forms of salts of Rucaparib of the present disclosure, can be used as medicaments, particularly for the treatment of cancer.

The present disclosure also provides methods of treating cancer comprising administering a therapeutically effective amount of any one or a combination of the solid state forms of Rucaparib and/or solid state forms of salts of Rucaparib of the present disclosure, or at least one of the above pharmaceutical compositions, to a subject in need of the treatment.

Having thus described the disclosure with reference to particular preferred embodiments and illustrative examples, those in the art can appreciate modifications to the disclosure as described and illustrated that do not depart from the spirit and scope of the disclosure as disclosed in the specification. The Examples are set forth to aid in understanding the disclosure but are not intended to, and should not be construed to limit its scope in any way.

### Powder X-ray Diffraction ("PXRD") - Method 1

Sample after being powdered in a mortar and pestle is applied directly on a silicon plate holder. The X-ray powder diffraction pattern was measured with Philips X'Pert PRO X-ray powder diffractometer, equipped with Cu irradiation source =1.54184 Ǻ (Ǻngström), X'Celerator (2.022° 2θ) detector.

Scanning parameters: angle range: 3-40 deg., step size 0.0167, time per step 37 s, continuous scan.

The described peak positions were determined without using silicon powder as an internal standard in an admixture with the sample measured.

### EXAMPLES

Starting material may be prepared according to the disclosure of U.S. Patent Publication No. 2019/0389871.

Rucaparib can be prepared according to methods known from the literature, for example U.S. Patent No. 6,495,541. A process for its preparation is described in U.S. Patent No. 7,323,562, as well as in Org. Process Res. Dev., 2012, 16 (12), pp 1897-1904.

Rucaparib form C can be prepared according to any one of the examples described in International Publication No. WO 2018/140377.

### Alternatively, Rucaparib form C can be prepared as follows:

Rucaparib Hydrochloride (19.41 grams) was suspended in methanol (90 ml). The reaction mixture was heated to a temperature of about 67°C and the pH was adjusted to 11.5 by addition of aqueous NaOH (2M) over a period of 120 minutes. The obtained reaction mixture was further stirred for 1 hour at a temperature of about 65°C to about 70°C. Then, water was added dropwise, in a volume to complete a total volume of water and aqueous NaOH (90ml). The obtained suspension was cooled to a temperature of about 20°C to 25°C over a period of about 2 hours. The cooled suspension was filtered off and washed with 2x18ml of methanol/water mixture (1/1) and then a third wash by 18 ml of water. The isolated material was dried at a temperature of about 50°C until constant mass. A sample was and analyzed by XRPD, and form C was obtained.

### Example 1. Preparation of Rucaparib Tosylate form VII

15.8 grams of Rucaparib base (Form C) was suspended in 300 ml of methanol at room temperature. 10.2 grams of p-toluenesulfonic acid monohydrate was added into the obtained suspension. Suspension was dissolved at 65 °C and slowly cooled to 0 °C in 3 hours while crystallization occurred. Obtained suspension was stirred for 30 minutes at 0 °C and vacuum filtered. Sample was washed with 50 ml of cooled methanol and then vacuum dried at 80 °C to constant mass. Obtained solid was analyzed by XRPD. Rucaparib Tosylate form VII was obtained.

### Example 2. Preparation of Rucaparib Tosylate form VIII

15.8 grams of Rucaparib base (Form C) was suspended in 250 ml of methanol and heated to 60 °C. 10.2 grams of p-toluenesulfonic acid monohydrate was dissolved in 50 ml of methanol and added into the obtained suspension of Rucaparib base. Suspension was dissolved at 60 °C and slow cooled to 45 °C. 0.5 grams of crystalline Rucaparib Tosylate was added into the obtained solution and stirred for 15 minutes at 45 °C. Suspension was cooled to 0°C in 2 hours and stirred for 1 hour. Obtained solid was vacuum filtered and washed with cooled methanol. Obtained solid was analyzed by XRPD. Rucaparib Tosylate form VIII was obtained.

## Claims

1. Crystalline Form VII of Rucaparib Tosylate **characterized by** an X-ray powder diffraction pattern having peaks at 4.3, 8.5, 10.3 and 22.7 degrees 2-theta ± 0.2 degrees 2-theta.

2. Crystalline Form VII of Rucaparib Tosylate according to Claim 1, **characterized by** an X-ray powder diffraction pattern having peaks at 4.3, 8.5, 10.3 and 22.7 degrees 2-theta ± 0.2 degrees 2-theta and also having any one, two, three or four additional peaks selected from the group consisting of 7.3, 11.8, 14.0 and 17.9 degrees 2-theta ± 0.2 degrees 2-theta.

3. Crystalline Form VII of Rucaparib Tosylate according to Claim 1 or Claim 2, which is **characterized by** an X-ray powder diffraction pattern as depicted in Figure 1.

4. Crystalline Form VII of Rucaparib Tosylate according to any of Claims 1 to 3, which is an anhydrous form.

5. Crystalline Form VII of Rucaparib Tosylate according to any of Claims 1 to 4, which contains: no more than 20%, no more than 10%, no more than 5%, no more than 2%, no more than 1%, no more than 0.5%, no more than 0.2%, no more than 0.1%, or 0% of any other crystalline forms of Rucaparib Tosylate.

6. Crystalline Form VII of Rucaparib Tosylate according to any of Claims 1 to 5, which contains: no more than 20%, no more than 10%, no more than 5%, no more than 2%, no more than 1%, no more than 0.5%, no more than 0.2%, no more than 0.1%, or 0% of amorphous Rucaparib Tosylate.

7. A pharmaceutical composition comprising crystalline Form VII of Rucaparib Tosylate according to any of Claims 1 to 6.

8. Use of a crystalline Form VII of Rucaparib Tosylate according to any of Claims 1 to 6, for the preparation of a pharmaceutical composition and/or pharmaceutical formulation, and optionally wherein the pharmaceutical formulation is an oral formulation, optionally wherein the oral formulation is a tablet or capsule, and particularly a tablet.

9. A pharmaceutical formulation comprising Form VII of Rucaparib Tosylate according to any of Claims 1 to 6, or a pharmaceutical composition of Claim 7, and at least one pharmaceutically acceptable excipient.

10. A process for preparing the pharmaceutical composition according to Claim 7 or a pharmaceutical formulation according to Claim 9, comprising combining a crystalline form VII of Rucaparib Tosylate according to any of Claims 1 to 6, with at least one pharmaceutically acceptable excipient.

11. Crystalline Form VII of Rucaparib Tosylate according to any of Claims 1 to 6, a pharmaceutical composition of Claim 7, or a pharmaceutical formulation according to Claim 9, for use as a medicament.

12. Crystalline Form VII of Rucaparib Tosylate according to any of Claims 1 to 6, a pharmaceutical composition of Claim 7, or a pharmaceutical formulation according to Claim 9, for use in the treatment of cancer.

13. Crystalline Form VII of Rucaparib Tosylate according any of Claims 1 to 6, a pharmaceutical composition of Claim 7, or a pharmaceutical formulation according to Claim 9, for the manufacture of a medicament for treatment of cancer.

14. Use of a crystalline Form VII of Rucaparib Tosylate according to any of Claims 1 to 6, in the preparation of another solid state form of Rucaparib Tosylate Rucaparib , or another Rucaparib salt, co-crystal or solid state form thereof.

15. A process for preparing an Rucaparib salt or a solid state form thereof comprising preparing crystalline Form VII of Rucaparib Tosylate according to any of Claims 1 to 6, and converting it to another Rucaparib salt, co-crystal or a solid state form thereof; and optionally further comprising combining the resulting Rucaparib salt, co-crystal or a solid state form thereof, with at least one pharmaceutically acceptable excipient to prepare a pharmaceutical composition or formulation.

## Patentansprüche

1. Kristalline Form VII von Rucaparibtosylat, **gekennzeichnet durch** ein Röntgenpulverbeugungsmuster mit Peaks bei 4,3, 8,5, 10,3 und 22,7 Grad 2-Theta ± 0,2 Grad 2-Theta.

2. Kristalline Form VII von Rucaparibtosylat nach Anspruch 1, **gekennzeichnet durch** ein Röntgenpulverbeugungsmuster mit Peaks bei 4,3, 8,5, 10,3 und 22,7 Grad 2-Theta ± 0,2 Grad 2-Theta sowie mit einem, zwei, drei oder vier zusätzlichen Peaks, ausgewählt aus der Gruppe bestehend aus 7,3, 11,8, 14,0 und 17,9 Grad 2-Theta ± 0,2 Grad 2-Theta.

3. Kristalline Form VII von Rucaparibtosylat nach Anspruch 1 oder Anspruch 2, die durch ein Röntgenpulverbeugungsmuster gemäß der Darstellung in Figur 1 gekennzeichnet ist.

4. Kristalline Form VII von Rucaparibtosylat nach einem der Ansprüche 1 bis 3, bei der es sich um eine wasserfreie Form handelt.

5. Kristalline Form VII von Rucaparibtosylat nach einem der Ansprüche 1 bis 4, die nicht mehr als 20 %, nicht mehr als 10 %, nicht mehr als 5 %, nicht mehr als 2 %, nicht mehr als 1 %, nicht mehr als 0,5 %, nicht mehr als 0,2 %, nicht mehr als 0,1 % oder 0 % jeglicher anderen kristallinen Formen von Rucaparibtosylat enthält.

6. Kristalline Form VII von Rucaparib-Tosylat nach einem der Ansprüche 1 bis 5, die nicht mehr als 20 %, nicht mehr als 10 %, nicht mehr als 5 %, nicht mehr als 2 %, nicht mehr als 1 %, nicht mehr als 0,5 %, nicht mehr als 0,2 %, nicht mehr als 0,1 % oder 0 % amorphes Rucaparibtosylat enthält.

7. Pharmazeutische Zusammensetzung, umfassend kristalline Form VII von Rucaparibtosylat nach einem der Ansprüche 1 bis 6.

8. Verwendung einer kristallinen Form VII von Rucaparibtosylat nach einem der Ansprüche 1 bis 6 zur Herstellung einer pharmazeutischen Zusammensetzung und/oder pharmazeutischen Formulierung, und gegebenenfalls wobei die pharmazeutische Formulierung eine orale Formulierung ist, gegebenenfalls wobei die orale Formulierung eine Tablette oder Kapsel und insbesondere eine Tablette ist.

9. Pharmazeutische Formulierung, umfassend Form VII von Rucaparibtosylat nach einem der Ansprüche 1 bis 6 oder eine pharmazeutische Zusammensetzung nach Anspruch 7 und mindestens einen pharmazeutisch unbedenklichen Hilfsstoff.

10. Verfahren zur Herstellung der pharmazeutischen Zusammensetzung nach Anspruch 7 oder einer pharmazeutischen Formulierung nach Anspruch 9, bei dem man eine kristalline Form VII von Rucaparibtosylat nach einem der Ansprüche 1 bis 6 mit mindestens einem pharmazeutisch unbedenklichen Hilfsstoff vereinigt.

11. Kristalline Form VII von Rucaparibtosylat nach einem der Ansprüche 1 bis 6, pharmazeutische Zusammensetzung nach Anspruch 7 oder pharmazeutische Formulierung nach Anspruch 9 zur Verwendung als Medikament.

12. Kristalline Form VII von Rucaparibtosylat nach einem der Ansprüche 1 bis 6, pharmazeutische Zusammensetzung nach Anspruch 7 oder pharmazeutische Formulierung nach Anspruch 9 zur Verwendung bei der Behandlung von Krebs.

13. Kristalline Form VII von Rucaparibtosylat nach einem der Ansprüche 1 bis 6, pharmazeutische Zusammensetzung nach Anspruch 7 oder pharmazeutische Formulierung nach Anspruch 9 zur Herstellung eines Medikaments zur Behandlung von Krebs.

14. Verwendung einer kristallinen Form VII von Rucaparibtosylat nach einem der Ansprüche 1 bis 6 bei der Herstellung einer anderen Festkörperform von Rucaparibtosylat, Rucaparib oder eines anderen Rucaparibsalzes, eines Cokristalls oder einer Festkörperform davon.

15. Verfahren zur Herstellung eines Rucaparibsalzes oder einer Festkörperform davon, bei dem man kristalline Form VII von Rucaparibtosylat nach einem der Ansprüche 1 bis 6 herstellt und sie in ein anderes Rucaparibsalz, einen Cokristall oder eine Festkörperform davon umwandelt; und bei dem man gegebenenfalls ferner das erhaltene Rucaparibsalz, den erhaltenen Cokristall oder eine Festkörperform davon mit mindestens einem pharmazeutisch unbedenklichen Hilfsstoff vereinigt, um eine pharmazeutische Zusammensetzung oder Formulierung herzustellen.

## Revendications

1. Forme cristalline VII du tosylate de rucaparib **caractérisée par** un diagramme de diffraction des rayons X sur poudre présentant des pics à 2-thêta de 4,3, 8,5, 10,3 et 22,7 degrés ± 2-thêta de 0,2 degré.

2. Forme cristalline VII du tosylate de rucaparib selon la revendication 1, **caractérisée par** un diagramme de diffraction des rayons X sur poudre présentant des pics à 2-thêta de 4,3, 8,5, 10,3 et 22,7 degrés ± 2-thêta de 0,2 degré et présentant également un, deux, trois ou quatre pics supplémentaires quelconques sélectionnés dans le groupe constitué par 2-thêta de 7,3, 11,8, 14,0 et 17,9 degrés ± 2-thêta de 0,2 degré.

3. Forme cristalline VII du tosylate de rucaparib selon la revendication 1 ou la revendication 2, qui est **caractérisée par** un diagramme de diffraction des rayons X sur poudre tel que représenté à la figure 1.

4. Forme cristalline VII du tosylate de rucaparib selon l'une quelconque des revendications 1 à 3, qui est une forme anhydre.

5. Forme cristalline VII du tosylate de rucaparib selon l'une quelconque des revendications 1 à 4, qui ne contient pas plus de 20 %, pas plus de 10 %, pas plus de 5 %, pas plus de 2 %, pas plus de 1 %, pas plus de 0,5 %, pas plus de 0,2 %, pas plus de 0,1 % ou qui contient 0 % de quelconques autres formes cristallines du tosylate de rucaparib.

6. Forme cristalline VII du tosylate de rucaparib selon l'une quelconque des revendications 1 à 5, qui ne contient pas plus de 20 %, pas plus de 10 %, pas plus de 5 %, pas plus de 2 %, pas plus de 1 %, pas plus de 0,5 %, pas plus de 0,2 %, pas plus de 0,1 % ou qui contient 0 % de tosylate de rucaparib amorphe.

7. Composition pharmaceutique comprenant la forme cristalline VII du tosylate de rucaparib selon l'une quelconque des revendications 1 à 6.

8. Utilisation d'une forme cristalline VII du tosylate de rucaparib selon l'une quelconque des revendications 1 à 6, pour la préparation d'une composition pharmaceutique et/ou d'une formulation pharmaceutique et éventuellement dans laquelle la formulation pharmaceutique est une formulation orale, éventuellement dans laquelle la formulation orale est un comprimé ou une capsule, et en particulier un comprimé.

9. Formulation pharmaceutique comprenant la forme cristalline VII du tosylate de rucaparib selon l'une quelconque des revendications 1 à 6, ou une composition pharmaceutique selon la revendication 7, et au moins un excipient pharmaceutiquement acceptable.

10. Procédé pour la préparation de la composition pharmaceutique selon la revendication 7 ou d'une formulation pharmaceutique selon la revendication 9, comprenant la combinaison d'une forme cristalline VII du tosylate de rucaparib selon l'une quelconque des revendications 1 à 6 avec au moins un excipient pharmaceutiquement acceptable.

11. Forme cristalline VII du tosylate de rucaparib selon l'une quelconque des revendications 1 à 6, composition pharmaceutique selon la revendication 7 ou formulation pharmaceutique selon la revendication 9, destinées à être utilisées comme médicament.

12. Forme cristalline VII du tosylate de rucaparib selon l'une quelconque des revendications 1 à 6, composition pharmaceutique selon la revendication 7 ou formulation pharmaceutique selon la revendication 9, destinées à être utilisées dans le traitement d'un cancer.

13. Forme cristalline VII du tosylate de rucaparib selon l'une quelconque des revendications 1 à 6, composition pharmaceutique selon la revendication 7 ou formulation pharmaceutique selon la revendication 9, pour la fabrication d'un médicament pour le traitement d'un cancer.

14. Utilisation d'une forme cristalline VII du tosylate de rucaparib selon l'une quelconque des revendications 1 à 6 dans la préparation d'une autre forme à l'état solide du tosylate de rucaparib ou d'un autre sel de rucaparib, d'un cocristal ou d'une forme à l'état solide correspondante.

15. Procédé pour la préparation d'un sel de rucaparib ou d'une forme à l'état solide correspondante, comprenant la préparation de forme cristalline VII du tosylate de rucaparib selon l'une quelconque des revendications 1 à 6 et la conversion de celle-ci en un autre sel de rucaparib, un cocristal ou une forme à l'état solide correspondante ; et éventuellement comprenant en outre la combinaison du sel de rucaparib, du cocristal résultants ou d'une forme à l'état solide correspondante avec au moins un excipient pharmaceutiquement acceptable pour préparer une composition ou formulation pharmaceutique.
